# EUROPEAN PATENT APPLICATION

(11) **EP 0 820 999 A1**
(43) Date of publication of application: **28.01.1998**
(21) Application number: 97107908.2
(22) Date of filing: 15.05.1997
(51) Int. Cl.: C07D 499/68

(54) **A process for the preparation of penicillins**

(30) Priority: 26.07.1996 IT MI961595
(71) Applicant: Ribbon S.r.L., 20145 Milano (IT)
(72) Inventor: Fortunato, Giuseppe, 20145 Milano (IT); Verzini, Massimo, 20145 Milano (IT); Boni, Riccardo, 20145 Milano (IT); Maruzzelli, Gennaro, 20145 Milano (IT)
(74) Representative: Minoja, Fabrizio

(57) **Abstract**

The present invention relates to a process for the preparation of penicillins and particularly for the preparation of 6-D-(-)-α-(4-ethyl-2,3-dioxo-1-piperazinylcarbonylamino)-α-phenylacetamidopenicillanic acid known under the trade name piperacillin.

## Description

### Field of the invention

The present invention relates to a process for the preparation of penicillins and particularly for the preparation of 6-D-(-)-α-(4-ethyl-2,3-dioxo-1-piperazinylcarbonylamino)-α-phenylacetamidopenicillanic acid known under the trade name piperacillin.

### Technological background

A series of piperacillin-like compounds belonging to a wider penicillins class having a marked anti-bacterial activity are described in literature. Among these penicillins, particularly interesting proved to be piperacillin, due to its antibacterial activity and favourable pharmacokynetic characteristics. The product is marketed as the sterile, freeze-dried sodium salt.

A number of patents exist involving the synthesis of piperacillin and derivatives thereof.

A first preparation method, described in patents: Japan Kokai 77 151 187, Japan Kokai 78 15 394 and Japan Kokai 78 44 584, comprises the reaction of α-(4-ethyl-2,3dioxo-1-piperazinecarbonyl)-amino-phenylacetic chloride (EPCP-Cl), (prepared in situ with PCl₅) with 6-aminopenicillanic acid. The method is complex both chemically and technologically, moreover it has to be carried out in strictly dry conditions and the recovery of the product is difficult. In the reaction conditions, markedly coloured reaction mixtures form, the recovery of the final product is complex, unpractical from the industrial point of view and onerous from the economic one.

A second alternative preparation method, described in patents: DE 3 627 581; DE 3 518 207; DD 237 166; Japan Kokai Tokkyo Koho JP 57 118 571; BE 892 370 and DE 3 208 506, involves the reaction of 4-ethyl-2,3-dioxopiperazinecarbonyl chloride with ampicillin in an aqueous-organic medium in the presence of bases.

Different solvent media are described, such as: water-CH₂Cl₂; water-ethyl acetate; water-acetone and the like. The most used bases are: NaHCO₃, NaOH and Et₃N. 4-Ethyl-2,3-dioxopiperazinecarbonyl chloride used as the intermediate in the preparation of piperacillin has a 70-75% maximum titre, is poorly stable and markedly coloured. This involves remarkable difficulties in the standardization of the process, with formation of coloured side-products difficult to remove.

The undesired colouration is transmitted to the final product, thus requiring long, complex, not always successful purification processes, to obtain a pharmaceutically acceptable compound.

A third alternative preparation method, described in ES 2 005 450, comprises reacting ampicillin and 4-ethyl-2,3-dioxopiperazinecarbonyl chloride activated with functional groups such as -OCOCH₃, OB₂ in organic solvents in the presence of diazabicycloundecene or silylating agents. This method as well has the drawback of using 4-ethyl-2,3-dioxopiperazinecarbonyl chloride as the intermediate, which has a low titre, is poorly stable and markedly coloured.

The recovery of the final product is complex, unpractical from the industrial point of view and onerous from the economic point of view, as long, complex purification processes are necessary to obtain a pharmaceutically acceptable compound.

### Summary

The Applicant has found a novel process which is the object of the present invention.

This process is easy to carry out on an industrial scale, allows to overcome the drawbacks of the known processes, gives a high-purity product in high yields.

Piperacillin is obtained by reacting 6APA with α-(4-ethyl-2,3-dioxo-1-piperazinecarbonyl)aminophenyl acetic acid monohydrate previously activated in an organic solvent.

The choice to use α-(4-ethyl-2,3-dioxo-1-piperazinecarbonyl)aminophenylacetic acid as the intermediate is due to the fact that this intermediate being stable and obtainable in highly pure forms. This allows to obtain, directly from the reaction, a highly pure final product which requires no further purifications and can immediately be transformed into the desired pharmaceutical form.

The following invention relates to a process for the preparation of piperacillin of formula 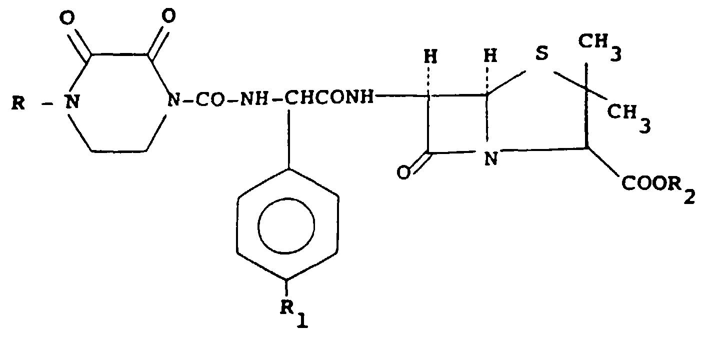 wherein
- R: is a straight or branched alkyl group, having 1 to 12 carbon atoms with 1 to 3 substituents selected from halogens and hydroxyl groups;
- R₁: is H; or OH; or a straight or branched alkyl group having 1 to 12 carbon atoms; or a phenyl group, optionally with 1 to 3 substituents selected from halogens and hydroxyl groups; a heterocyclic ring of 4 to 7 carbon atoms both aromatic and non aromatic, or a group selected from dihydrophenyl, cyclohexyl, hydroxymethyl, formamido, ureido and carboxymethylamino;
- R₂: is H; alkali or alkaline-earth metal cations, such as sodium, potassium and calcium, or other cations such as ammonium and the cations of organic amines.

The process of the invention comprises the following steps:
a) preparing a reactive mixed anhydride by reacting α-(4ethyl2,3-dioxopiperazinecarbonyl)aminophenyl acetic acid and alkyl chloroformate in an organic solvent 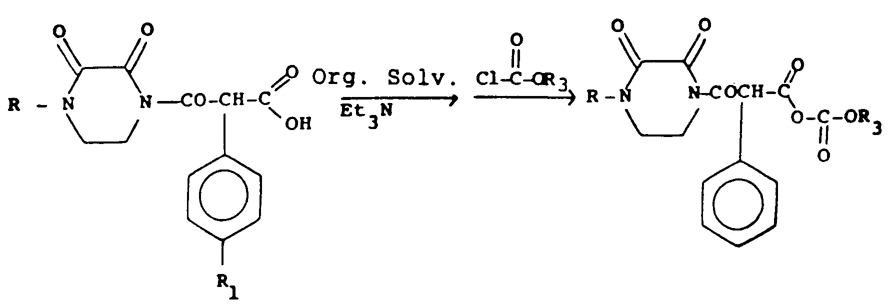 wherein R and R₁ have the meanings defined above and R₃ is a straight or branched alkyl group having 1 to 12 carbon atoms with 1 to 3 substituents selected from halogens or hydroxy groups;
b) reacting the mixed anhydride with 6-aminopenicillanic acid (6APA) in water/organic solvent medium to give the final compound at a pH ranging from 4 to 7 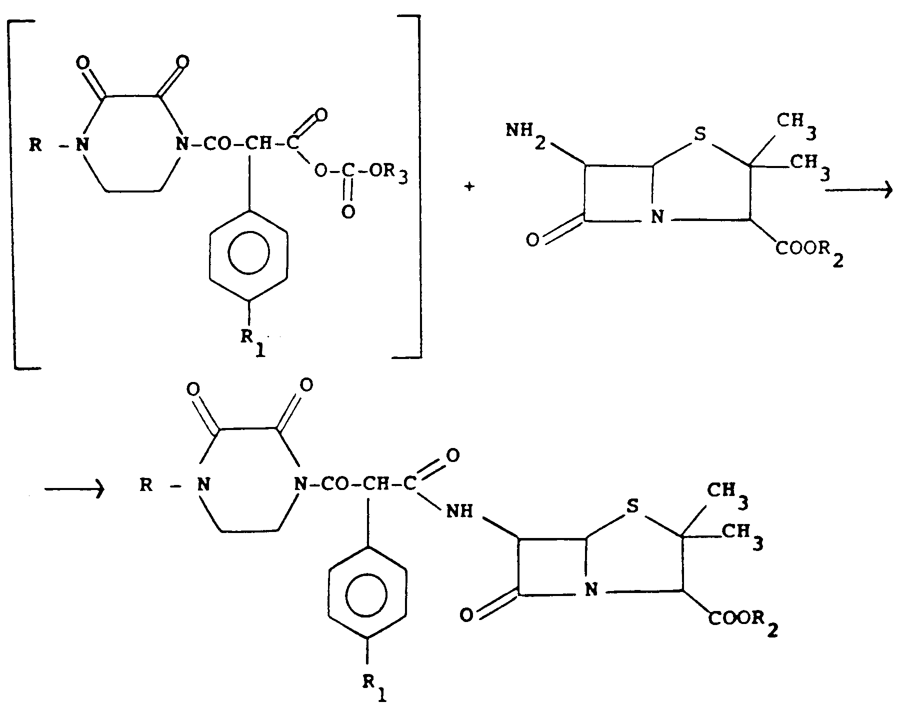 wherein R, R₁, R₂ and R₃ have the meanings defined above.
c) removing the reaction organic solvent by concentration under vacuum or by extraction with an organic solvent.
d) adding the precipitating solvent to the aqueous phase and precipitating piperacillin by adjusting pH to values ranging between 1.5 and 3.0

### Detailed disclosure

The intermediates used, 6-aminopenicillanic acid and α-(4-ethyl-2,3-dioxo-1-piperazinecarbonyl)aminophenylacetic acid are known intermediates, which are commercially available and anyhow widely described in literature and therefore easy to prepare.

In the first step, in which the α-(4-ethyl-2,3-dioxo-1-piperazinecarbonyl)aminophenylacetic acid mixed anhydride is prepared, the solvent is selected from toluene, ethyl acetate, methyl acetate, methylisobutylketone, acetone, methylene chloride and the proton acceptor is an inorganic or organic base selected from NaHCO₃, Na₂CO₃, Et₃N, tetramethylguanidine and diazobicycloundecene.

The mixed anhydride is prepared reacting the salified α-(4-ethyl-2,3-dioxo-1-piperazinecarbonyl)aminophenylacetic acid, in the above mentioned solvent media, with reactives such as pivaloyl chloride, ethyl chloroformate, butyl chloroformate.

The reaction temperature can range between -40°C and +15°C, temperatures between -30°C and +5°C being preferred.

The preparation of the α-(4-ethyl-2,3-dioxo-1-piperazinecarbonyl)aminophenylacetic acid mixed anhydride is carried out in a single step in mild reaction conditions which allow to significantly reduce the formation of side-products and coloured impurities which on the contrary are present, as already mentioned, in the known processes used for the preparation of piperacillin.

The reaction starts from the preparation of a compound of formula 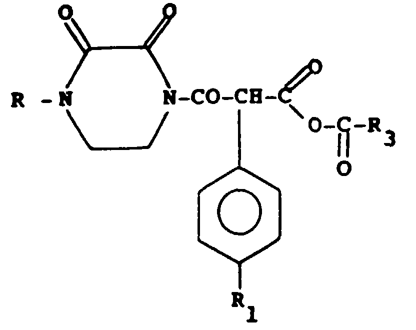 wherein R and R₁ have the meanings defined above and R₃ is preferably an alkyl group selected from ethyl, butyl, isopropyl and isobutyl.

The compound is not recovered and is used in situ in the reaction medium.

The subsequent step consists in reacting the mixed anhydride with 6APA dissolved in a solvent such as: acetone, ethyl acetate, methyl acetate, methyl isobutyl ketone, water and methylene chloride and the proton acceptor is an inorganic or organic base selected from NaHCO₃, Na₂CO₃, Et₃N, Et₂NH, EtNH₂, NaOH, KOH, LiOH, diazobicycloundecene.

The reaction is performed by condensation of 6APA with the mixed anhydride in the solvent media mentioned above at a temperature ranging between -40°C and +5°C.

The reaction has a high conversion degree thus allowing to reduce remarkably the reaction side-products and the coloured impurities.

As a reaction product, a compound of formula 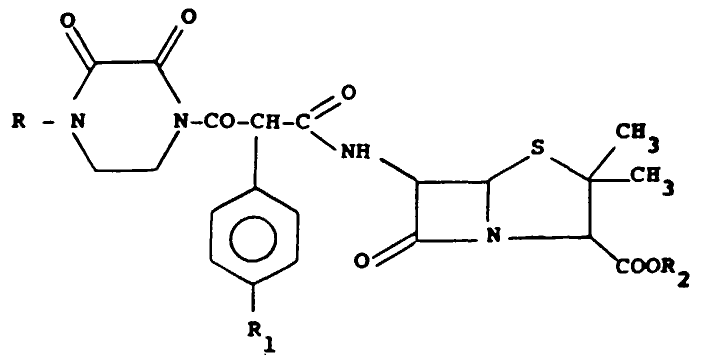 wherein R, R₁ and R₂ have the above mentioned meanings, is obtained.

The subsequent step is the recovery of piperacillin from the reaction medium.

The final compound is extracted with water and the solvent used as the reaction medium is removed by concentration under vacuum or by extraction with a solvent.

The concentration is performed at 30-70°C under vacuum and the solvent content in the residual aqueous phase should be below 5-10%.

Another method to remove the reaction solvent consists in extracting it from the aqueous mixture using a different solvent.

For this extraction, a solvent a selected from methylene chloride, ethyl acetate, toluene and methyl isobutyl ketone can be used. The solvent final content in the aqueous phase containing the product should be below 5-10%.

The latter extraction method is convenient in that is faster, easy to perform industrially and it substantially decreases the formation of coloured impurities due to the concentration under vacuum.

At the end of this operation, piperacillin is in the aqueous phase at a pH from 4.0 to 7.0 and the solution is clarified by treatment with active charcoal.

The aqueous solution is added with a solvent which can be selected from methyl isobutyl ketone, methyl acetate, THF and ethyl acetate, and piperacillin is precipitated under stirring by adjusting pH between 1 and 3 with hydrochloric acid. Among these solvents, ethyl acetate is preferred.

The precipitated crystalline white product is filtered, washed with water and dried under vacuum at temperatures of 20-60°C to a residual water content below 5%.

The method for the preparation of piperacillin herein described, in addition to the advantages mentioned above, is particularly convenient from the industrial point of view. The final product is obtained directly from the reaction mixture by precipitation, has a high chemical purity degree and the residual solvents contained therein can easily be removed with conventional techniques.

The following examples further illustrate the present invention.

### Example 1

320 ml of acetone are added with 43.8 g of α-(4-ethyl-2,3-dioxo-1-piperazinecarbonyl)aminophenylacetic acid and 19.5 ml of triethylamine. The mixture is cooled at -25°C and 13.6 ml of ethyl chloroformate and 0.8 ml of N-methylmorpholine are added thereto.

The mixture is reacted for 60 minutes at -25°C. The resulting suspension is added quickly with 30 g of 6APA dissolved in 60 ml of H₂O with 20% sodium hydroxide. The mixture is reacted for 30 minutes a -20°C. The reaction solution is added with 100 ml of H₂O and 300 ml of methylene chloride. The phases are separated, the aqueous phase containing the compound is added with 2.5 g of active charcoal and filtered. The filtered aqueous solution is added with 300 ml of ethyl acetate and the compound is precipitated at 20°C adjusting pH to 2.0 with concentrated hydrochloric acid.

After 1 hour the compound is filtered, washed with water and dried: 55 g of acid piperacillin are obtained.
Yield: 77%
HPLC titre: 99.0%
¹H-NMR (pyridine d₅): 1.10 (t, J = 7.1 Hz, 3H); 1.50 (s, 3H); 1.65 (s, 3H); 3.41 (m, 4H); 4.03 (m, 2H); 4.50 (s, 1H); 5.69 (d, J = 4.3 Hz, 1H); 6.18 (dAB, J = 4.3, 8.1 Hz, 1H); 6.24 (d, J = 7.1 Hz, 1H); 7.33-7.44 (m, 3H); 7.65-8.30 (m, 3H); 10.59 (d, J = 8.1 Hz, 1H); 10.61 (d, J = 7.1 Hz, 1H).

### Example 2

300 ml of methyl acetate are added with 43.8 g of α-(4-ethyl-2,3-dioxo-1-piperazinecarbonyl)aminophenylacetic acid and 19.5 ml of triethylamine. The mixture is cooled at -35°C and added with 13.6 ml of ethyl chloroformate and 0.8 ml of lutidine. The mixture is reacted at -35°C for 60 minutes.

The resulting suspension is added quickly with 30 g of 6APA dissolved in 60 ml of H₂O with triethylamine.

The mixture is reacted for 30 minutes at -35°C. The reaction solution is added with 100 ml of H₂O and 300 ml of ethyl acetate. The phases are separated and the aqueous phase containing the compound is added with 2.5 g of active charcoal and filtered.

The filtered aqueous solution is added with 300 ml of ethyl acetate: at 20°C the compound is precipitated adjusting pH to 2.0 with concentrated hydrochloric acid.

After 1 hour the compound is filtered, washed with water and dried: 53 g of acid piperacillin are obtained.
Yield: 74%
HPLC titre: 98%
¹H-NMR: (pyridine d₅) ppm: 1.05 (t, J = 7.1 Hz, 3H); 1.46 (s, 3H); 1.60 (s, 3H); 3.38 (m, 4H); 3.98 (m, 2H); 4.46 (s, 1H); 5.65 (d, J = 4.2 Hz, 1H); 6.12 (dAB, J = 4.2-8.1 Hz, 1H); 6.20 (d, J = 7.1 Hz, 1H); 7.30-7.40 (m, 3H); 7.60-8.24 (m, 3H); 10.54 (d, J = 8.1 Hz, 1H); 10.57 (d, J = 7.1 Hz, 1H).

### Example 3

300 ml of acetone are added with 43.8 g of α-(4-ethyl-2,3-dioxo-1-piperazinecarbonyl)aminophenylacetic acid and 19.5 ml of triethylamine. The mixture is cooled at -30°C and added with 17.2 ml of butyl chloroformate and 0.8 ml of N-methylmorpholine. The mixture is reacted at -35°C for 60 minutes.

The resulting suspension is added quickly with 30 g of 6APA dissolved in 60 ml of H₂O with triethylamine.

The mixture is reacted for 30 minutes at -30°C.

The reaction solution is added with 100 ml of H₂O and 300 ml of toluene. The phases are separated and the aqueous phase containing the compound is added with 2 g of active charcoal and filtered. The filtered aqueous solution is added with 300 ml of ethyl acetate: at 20°C the compound is precipitated adjusting pH to 2.0 with concentrated hydrochloric acid. After 2 hours the compound is filtered, washed with water and dried: 58 g of acid piperacillin are obtained.
Yield: 81%
HPLC titre: 100%
¹H-NMR: (pyridine d₅) ppm: 1.12 (t, J = 7.0 Hz, 3H); 1.51 (s,3H); 1.67 (s, 3H); 3.42 (m, 4H); 4.04 (m, 2H); 4.50 (s, 1H); 5.70 (d, J = 4.2 Hz, 1H); 6.20 (dAB, J = 4.2-8.0 Hz, 1H); 6.25 (d, J = 7.0 Hz, 1H); 7.32-7.45 (m, 3H); 7.65-8.28 (m, 3H); 10.60 (d, J = 8.0 Hz, 1H); 10.62 (d, J = 7.0 Hz, 1H).

## Claims

1. A process for the preparation of penicillins of formula 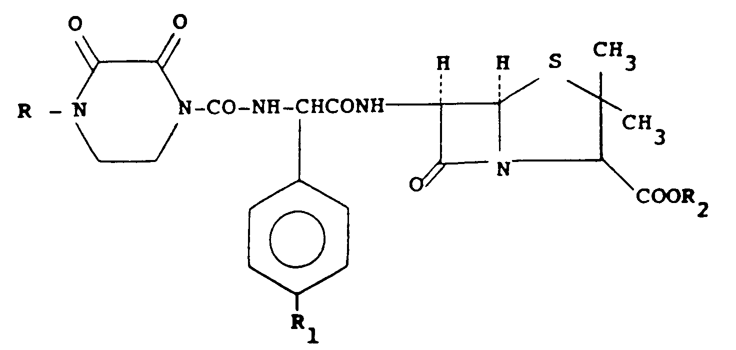 wherein
R is a straight or branched alkyl group, having 1 to 12 carbon atoms with 1 to 3 substituents selected from halogens and hydroxy groups;
R₁ is H; or OH; or a straight or branched alkyl group having 1 to 12 carbon atoms; or a phenyl group, optionally with 1 to 3 substituents selected from halogens and hydroxy groups; a heterocyclic ring of 4 to 7 carbon atoms both aromatic and non aromatic, or a group selected from dihydrophenyl, cyclohexyl, hydroxymethyl, formamido, ureido and carboxymethylamino;
R₂ is H; alkali or alkaline-earth metal cations, such as sodium, potassium and calcium, or other cations such as ammonium and the cations of organic amines;
which comprises the following steps:
a) preparing a reactive mixed anhydride by reaction between α-(4-ethyl-2,3-dioxopiperazinecarbonyl)-aminophenyl acetic acid and compounds of formula 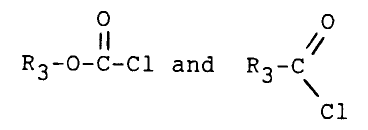 in organic solvents selected from toluene, ethyl acetate, methyl isobutyl ketone, acetone and methylene chloride and in the presence of proton acceptors selected from NaHCO₃, Na₂CO₃, Et₃N, TMG and diazobicycloundecene to obtain a compound of formula 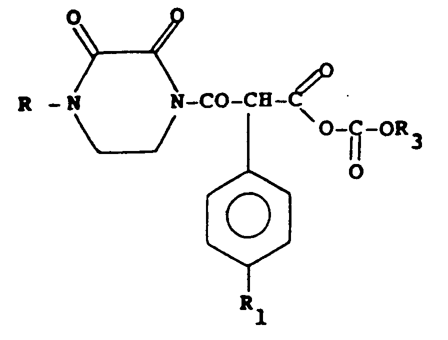 wherein R and R₁ have the above mentioned meanings and R₃ is a straight or branched alkyl group having 1 to 12 carbon atoms with 1 to 3 substituents selected from halogens or hydroxyl groups;
b) reacting the mixed anhydride with 6-aminopenicillanic acid (6APA) or derivatives thereof in an organic phase or a water-solvent solution to give a compound of formula 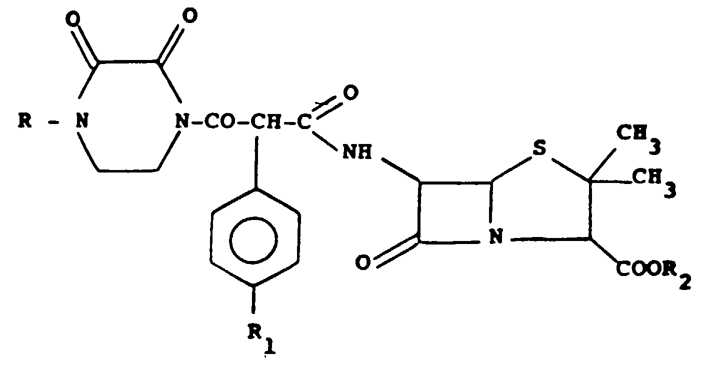 wherein R, R₁ and R₂ have the meanings defined above.

2. A process according to claim 1, wherein the final product is extracted in an aqueous phase and the reaction solvent is removed by concentration under vacuum or by differential extraction with solvents selected from methylene chloride, ethyl acetate, toluene and methyl isobutyl ketone.

3. A process according to claim 1 or 2, wherein the final product is precipitated in a water-solvent mixture adjusting pH with acids at values ranging from 0.5 to 3, using methyl isobutyl ketone, methyl acetate, tetrahydrofuran, ethyl acetate or acetone as solvents.

4. A process as claimed in claim 1, wherein step a) is carried out at temperatures ranging between -45° and +5°C.

5. A process as claimed in claim 1, wherein step a) is carried out in solvents such as toluene, ethyl acetate, methyl acetate, methyl isobutyl ketone and methylene chloride.

6. A process as claimed in claim 1, wherein step a) is carried out reacting α-(4-ethyl-2,3-dioxo-1-piperazinecarbonyl)aminophenylacetic acid with reactive chlorides of formula 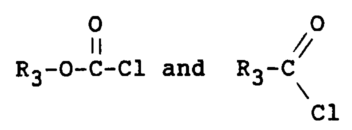 wherein R₃ is an alkyl group selected from ethyl, butyl, isopropyl and isobutyl.

7. A process as claimed in claim 1, wherein step b) is carried out in organic phase or in a mixture of water and a solvent selected from toluene, ethyl acetate, methyl acetate, methyl isobutyl ketone, acetone or methylene chloride.

8. A process as claimed in claim 1, wherein step b) is carried out reacting the mixed anhydride with 6APA dissolved in an organic solvent selected from toluene, ethyl acetate, methyl acetate, methyl isobutyl ketone, acetone or methylene chloride by means of silylation or using organic bases selected from Et₃N, Et₂NH, tetramethylguanidine, diazobicycloundecene.

9. A process as claimed in claim 1, wherein step b) is carried out reacting the mixed anhydride with 6APA dissolved in water with bases selected from NaHCO₃, Na₂CO₃, Et₃N, Et₂NH, EtNH₂, NaOH, KOH, LiOH, diazobicycloundecene.

10. A process as claimed in claim 1, wherein step c) is carried out by concentration under vacuum at a temperature ranging between 10°C and 70°C.

11. A process as claimed in claim 1, wherein step c) is carried out by differential extraction using toluene, ethyl acetate, methyl acetate, methyl isobutyl ketone, acetone and methylene chloride as solvents.

12. A process as claimed in claim 1, wherein step d) is carried out adjusting pH to values ranging between 0.5 and 3.0 with strong acids.

13. A process as claimed in claim 1, wherein step d) is carried out in a mixture of water and a solvent selected from toluene, ethyl acetate, methyl acetate, methyl isobutyl ketone, acetone and methylene chloride.
